# EUROPEAN PATENT APPLICATION

(11) **EP 3 677 225 A1**
(43) Date of publication of application: **08.07.2020**
(21) Application number: 18851127.3
(22) Date of filing: 08.08.2018
(51) Int. Cl.: A61F 2/36

(54) **STEM, FEMORAL COMPONENT, AND ARTIFICIAL HIP JOINT**

(30) Priority: 28.08.2017 JP 2017162995
(71) Applicant: Nantoh. Co., Ltd, Numazu-shi, Shizuoka 410-0301 (JP)
(72) Inventor: ISHIWATA, Teruo, Numazu-shi Shizuoka 410-0301 (JP)
(74) Representative: Grünecker Patent- und Rechtsanwälte PartG mbB
(86) International application number: PCT/JP2018/029704
(87) International publication number: WO 2019/044433

(57) **Abstract**

A stem (3) includes: a body section (10) which is inserted into a narrowing hole (Ha) formed in a femur (H) and osseointegrated; a neck section (20) which is joined to a tip of the body section (10) and protrudes from the narrowing hole (Ha) to transmit a load from an acetabular side to the body section (10); and a leg section (25) which is joined to a distal end of the body section (10) to hold a posture of the body section (10), wherein the neck section (20) and the leg section (25) are made of a biocompatible resin, and the body section (10) is made of a biocompatible metal, a biocompatible ceramic, or a biocompatible resin.

## Description

### Technical Field

The present invention relates to a stem, a femoral component, and a hip prosthesis.

### Background Art

Implants for body implantation have attracted attention. Implants include dental implants for jaw bone implantation, as well as artificial joint implants for femoral implantation or the like.

An artificial joint is an artificial member which can replace a joint for recovering functions of the joint when the joint is damaged due to diseases, wounds, or the like. In Japan and the like, demand for artificial joints is increasing with the progress in the aging society. For this reason, the number of artificial joint replacement surgery is increasing year by year.

Among artificial joints, a hip prosthesis applied to a hip joint is composed of a femoral component implanted into a femur, and an acetabular component implanted into an acetabulum.

The femoral component has a stem and a head. The head plays a role of a femoral head, and the stem is implanted into a femur to support the head.

The acetabular component has a cup and a liner. The liner plays a role of an articular surface of an acetabulum, and the cup is implanted into the acetabulum to support the liner. The liner is also referred to as an acetabular shell, and the cup is also referred to as a socket.

The stem, the head, and the cup are formed of a metal such as a titanium alloy and a cobalt-chromium alloy, as well as a ceramic such as alumina and zirconia.

Of the hip prosthesis, the stem and the cup are implanted into bone. The cup is fixed to a concave area of an acetabular cartridge, and the stem is fixed to a hole which is formed so as to reach a medullary cavity.

The methods for fixing the stem to a femur includes two methods, a cement method and a cementless method.

In the cementless method, a surface of the stem is subjected to special processing for osseointegration between the stem and the femur. That is, biological fixability is obtained by bone ingrowth.

### Prior Art Documents

### Patent Documents

Patent Document 1: JP2014-226265A

### Summary of Invention

### Problem to be solved

A femoral component (such as stem) is extremely expensive because it is made of alloys or ceramics. The stem has a specific gravity and a rigidity (Young's modulus) which are higher than those of human bone (cortical bone). A specific gravity of human bone is about 2.0 while specific gravities of a titanium alloy, a cobalt-chromium alloy, and a zirconia alloy are about 4.5, about 8.8, and about 6.0, respectively. Human bone has a Young's modulus of 10 to 30 GPa, and on the other hand, the titanium alloy has a Young's modulus of about 110 GPa, the cobalt-chromium alloy has a Young's modulus of about 210 GPa, and the zirconia has a Young's modulus of about 200 GPa.

For this reason, when a stem is implanted into a hip joint, a femoral region has leadenness, uncomfortable feeling, dull pain, pain, or the like in some cases. In particular, when the stem is implanted into only one of hip joints, the uncomfortable feeling tends to increase.

Even if a surface of a stem or the like is subjected to special processing, it takes several weeks to several months for the stem or the like to be osseointegrated. If an excessive force is applied to the stem or the like during this period, the surrounding bones or the like may be damaged, osseointegration is delayed, or osseointegration becomes difficult. Osseointegration of the stem or the like should be improved for reducing burdens on patients.

Even if the stem is sufficiently osseointegrated, bone atrophy due to stress shielding may be caused as a long period of time elapses.

As the stress shielding progresses, the stem may be loosened, or the femur may be weakened, and therefore the bone is more likely to be broken. This causes a risk that the long-term stability of the stem is adversely affected. Thus, for the stem, the bone atrophy due to the stress shielding should be minimized.

It has been studied to form the stem with a biocompatible resin such as a polyetheretherketone resin. The biocompatible resin is lightweight and has a Young's modulus of about 1 to 5 GPa, and therefore, even when the stem is implanted, uncomfortable feeling or the like hardly occur. Since the stem made of the biocompatible resin leads to small stress shielding, a load on the femur is sufficiently distributed, and the bone atrophy can be reduced.

However, there is a problem that the stem made of the biocompatible resin is hard to osseointegrate as it is. Furthermore, since the stem is elongated, if the stem is formed of a biocompatible resin, the stem may be broken by a high torsional load.

An object of the present invention is to provide a stem, a femoral component, and a hip prosthesis which can reduce an uncomfortable feeling after implantation and can reduce bone atrophy due to stress shielding.

### Solution to Problem

A first embodiment of the stem according to the present invention is characterized in that it includes: a body section which is inserted into a narrowing hole formed in a femur and osseointegrated; a neck section which is joined to a tip of the body section and protrudes from the narrowing hole to transmit a load from an acetabular side to the body section; and a leg section which is joined to a distal end of the body section to hold a posture of the body section, the neck section and the leg section being made of a biocompatible resin, and the body section being made of a biocompatible metal, a biocompatible ceramic, or a biocompatible resin.

A second embodiment of the stem according to the present invention is characterized in that, in the first embodiment, the biocompatible resin is a polyetheretherketone resin.

A third embodiment of the stem according to the present invention is characterized in that, in the first or second embodiment, the biocompatible metal is a titanium alloy or a cobalt-chromium alloy.

A fourth embodiment of the stem according to the present invention is characterized in that, in the first or second embodiment, the biocompatible ceramic contains zirconia.

A fifth embodiment of the stem according to the present invention is characterized in that, in any one of the first to fourth embodiments, an entire length of the body section is 40% or more and 60% or less of an entire length of the leg section.

A sixth embodiment of the stem according to the present invention is characterized in that, in any one of the first to fifth embodiments, the body section has a hollow space accounting for 40% or more and 60% or less of an entire volume.

A seventh embodiment of the stem according to the present invention is characterized in that, in any one of the first to sixth embodiments, an outer surface of the body section has a biological tissue-adhering face on which numerous fingertip-shaped villi are formed.

An eighth embodiment of the stem according to the present invention is characterized in that, in the seventh embodiment, the villi on the biological tissue-adhering face have a tip diameter in the order of nanometers.

A ninth embodiment of the stem according to the present invention is characterized in that, in the eighth embodiment, the villi on the biological tissue-adhering face have a tip diameter of 50 nm or more and less than 500 nm.

A tenth embodiment of the stem according to the present invention is characterized in that, in any one of the seventh to ninth embodiments, the biological tissue-adhering face has a three-dimensional surface roughness Sa in the order of nanometers.

An eleventh embodiment of the stem according to the present invention is characterized in that, in any one of the seventh to tenth embodiments, an interface of the biological tissue-adhering face has a developed area ratio Sdr of 0.1 or more and 2.0 or less.

A twelfth embodiment of the stem according to the present invention is characterized in that, in any one of the seventh to eleventh embodiments, the biological tissue-adhering face has many first grooves having a width of 1 µm or more and 50 µm or less.

A thirteenth embodiment of the stem according to the present invention is characterized in that, in the twelfth embodiment, the first grooves have a depth of 0.1µm or more and 10 µm or less.

A fourteenth embodiment of the stem according to the present invention is characterized in that, in any one of the seventh to thirteenth embodiments, the biological tissue-adhering face has many second grooves having a width of 10 µm or more and 500 µm or less.

A fifteenth embodiment of the stem according to the present invention is characterized in that, in fourteenth embodiment, the second grooves have a depth of 2 µm or more and 100 µm or less.

A sixteenth embodiment of the stem according to the present invention is characterized in that, in any one of the seventh to fifteenth embodiments, the biological tissue-adhering face is formed through a laser nonthermal processing carried out by emitting a laser beam in air.

A first embodiment of the femoral component according to the present invention is characterized in that it includes: the stem according to any one of the first to sixteenth embodiments; and a spherical head attached to a tip of the stem and receiving a load from an acetabular side, the head including an inner sphere made of a biocompatible resin and joined to the neck section, and an outer sphere made of a biocompatible metal or a biocompatible ceramic and surrounding the inner sphere.

A second embodiment of the femoral component according to the present invention is characterized in that, in the first embodiment, the outer sphere includes a plurality of disc bodies having a spherical segment shape or an end spherical segment shape, and is configured by joining the plurality of disc bodies.

A first embodiment of the hip prosthesis according to the present invention is characterized in that it includes: the femoral component according to the first or second embodiment; and an acetabular component having a cup and a liner and in close contact with the femoral component.

The second embodiment of the hip prosthesis according to the present invention is characterized in that, in the first embodiment, an outer surface of the cup has a biological tissue-adhering face on which numerous fingertip-shaped villi are formed.

### Effects of Invention

Since the stem, the femoral component, and the hip prosthesis according to the present invention have a specific gravity and a rigidity approximate to those of human bone, leadenness, uncomfortable feeling, dull pain, pain, or the like of a femoral region can be reduced. In addition, since the stem, the femoral component, and the hip prosthesis according to the present invention have the rigidity approximate to that of human bone, bone atrophy due to a stress shielding can be reduced. Furthermore, high level of osseointegration can be achieved in a shorter period than before.

### Brief Description of Drawings

Figure 1 is a diagram illustrating a hip prosthesis 1 according to an embodiment of the present invention.
Figure 2 is a perspective view illustrating a femoral component 2 according to an embodiment of the present invention.
Figure 3 is an exploded perspective view illustrating a stem 3 according to the first embodiment of the present invention.
Figure 4 (a) is a longitudinal sectional view of a head 4.
Figure 4 (b) is a bottom view of the head 4.
Figure 5 (a) is a longitudinal sectional view of an inner sphere 30.
Figure 5 (b) is a bottom view of the inner sphere 30.
Figure 6 is an exploded perspective view illustrating the head 4.
Figure 7 is photographs of a biological tissue-adhering face 51 taken by SEM at (a) a magnification of 200 times, (b) a magnification of 500 times, and (c) a magnification of 2000 times.
Figure 8 is photographs of a biological tissue-adhering face 51 taken by SEM at (d) a magnification of 5000 times, and (e) a magnification of 10000 times.
Figure 9 is a reference photograph showing microvilli in a small intestine.
Figure 10 is photographs of outer surfaces of conventional stems taken by SEM (magnification: 2,000 times), including (a) a product manufactured by Company A, (b) a product manufactured by Company B, (c) a product manufactured by Company C, and (d) a product manufactured by Company D.
Figure 11 is photographs of a biological tissue-adhering face 52 taken by SEM at (a) a magnification of 200 times, (b) a magnification of 500 times, and (c) a magnification of 2000 times.
Figure 12 is photographs of a biological tissue-adhering face 52 taken by SEM at (d) a magnification of 5000 times, and (e) a magnification of 10000 times.
Figure 13 is photographs of a biological tissue-adhering face 53 taken by SEM at (a) a magnification of 200 times, (b) a magnification of 500 times, and (c) a magnification of 10000 times.
Figure 14 is photographs of a biological tissue-adhering face 54 taken by SEM at (a) a magnification of 500 times, and (b) a magnification of 10000 times.
Figure 15 is an exploded perspective view illustrating the body section 80.
Figure 16 is an exploded perspective view illustrating the stem 103 according to the second embodiment of the present invention.

### Description of Embodiments

Embodiments of the present invention will be explained with reference to the figures. Various dimensions and the like described in the following explanation are merely examples.

### [Hip Prosthesis 1, Femoral Component 2]

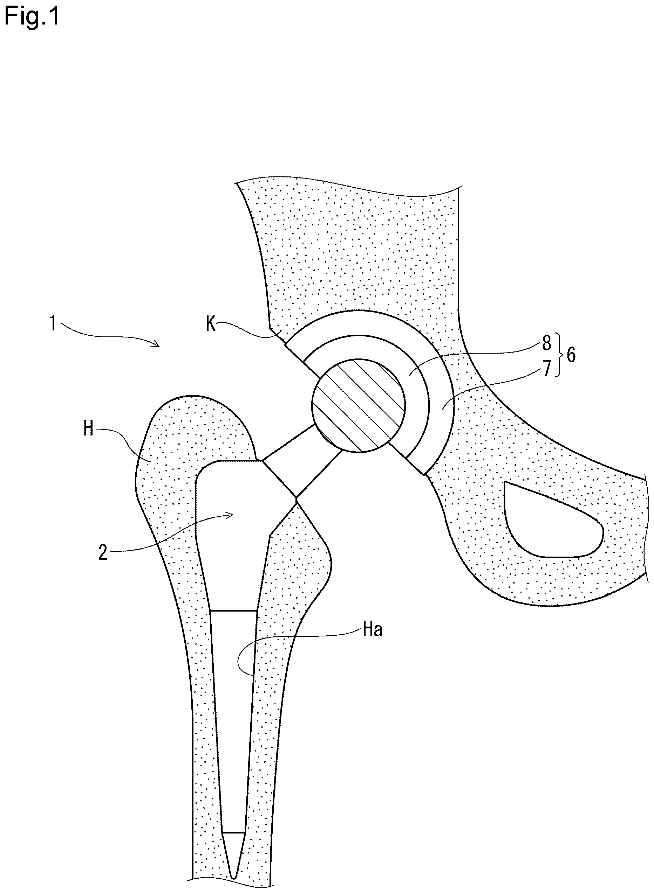

Fig. 1 is a diagram illustrating a hip prosthesis 1 according to an embodiment of the present invention.

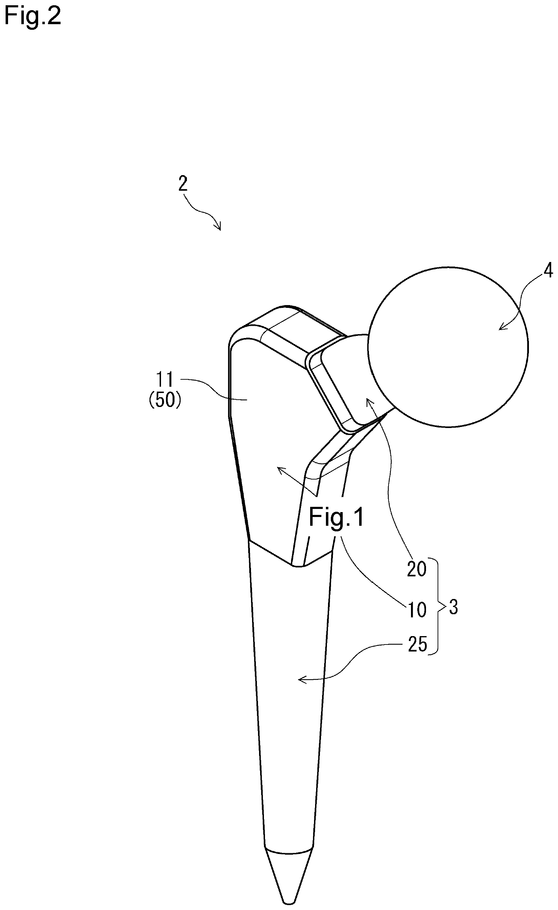

Fig. 2 is a perspective view illustrating a femoral component 2 according to an embodiment of the present invention.

The hip prosthesis 1 replaces a hip joint for recovering functions of the hip joint when the hip joint is damaged.

The hip prosthesis 1 consists of the femoral component 2 implanted into a femur H, and an acetabular component 6 implanted into an acetabulum K.

The femoral component 2 has a stem 3 and a head 4. The head 4 plays a role of an epiphysis of the femur H, and the stem 3 is implanted into the femur H to support the head 4.

The acetabular component 6 has a cup 7 and a liner 8. The liner 8 plays a role of an articular surface of the acetabulum K, and the cup 7 is implanted into the acetabulum K to support the liner 8.

An extending direction of the femoral component 2 is referred to as a vertical direction or a lengthwise direction. In the vertical direction, a side of the head 4 is referred to as a tip (first end), and a side of the stem 3 is referred to as a distal end (second end).

A width direction of the stem 3 is referred to as a lateral direction or a horizontal direction. A thickness direction of the stem 3 is referred to as an anteroposterior direction.

### [Stem 3]

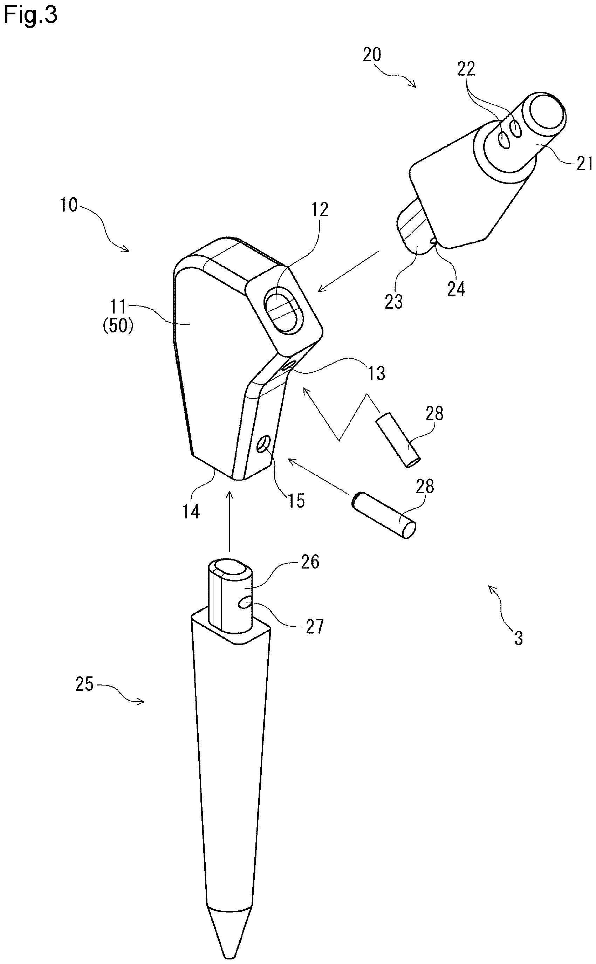

Fig. 3 is an exploded perspective view illustrating the stem 3 according to the first embodiment of the present invention.

The stem 3 includes a body section 10, a neck section 20, and a leg section 25.

The body section 10 is inserted into a narrowing hole Ha formed in the femur H and is osseointegrated. The body section 10 supports the neck section 20 and transmits a load to the femur H.

The neck section 20 is joined to the tip of the body section 10 and protrudes from the narrowing hole Ha to introduce the load from the acetabular side.

The leg section 25 is joined to the distal end of the body section 10 to hold a posture of the implanted body section 10. The leg section 25 guides the insertion of the body section 10 into the narrowing hole Ha.

The body section 10 is a block-shaped member extending in the vertical direction and has a length of about 50 mm. The body section 10 has a width gradually narrowing from the tip toward the distal end. The tip side has a width of about 33 mm and the distal end side has a width of about 15 mm. The body section 10 has a thickness which is substantially constant from the tip side toward the distal end side. The tip side has a thickness of about 13 mm and the distal end side has a thickness of about 11 mm. The body section 10 is made of a biocompatible ceramic. The biocompatible ceramic is e.g., zirconia.

The body section 10 has a neck-joining hole 12 on a tip face, and a leg-joining hole 14 on a distal end face. The tip face of the body section 10 is oriented at about 50° with respect to the vertical direction.

The neck-joining hole 12 is a substantially elliptical hole into which the neck section 20 is fitted. A circular lateral hole 13 orthogonally communicates with the neck-joining hole 12. The neck-joining hole 12 is exposed (opened) to the narrowing hole Ha when the body section 10 is inserted into the narrowing hole Ha.

The leg-joining hole 14 is a substantially elliptical hole into which the leg section 25 is fitted. A circular lateral hole 15 orthogonally communicates with the leg-joining hole 14. The leg-joining hole 14 faces to a bottom of the narrowing hole Ha when the body section 10 is inserted into the narrowing hole Ha.

The body section 10 is inserted into the narrowing hole Ha so that an outer surface 11 is osseointegrated with the femur H. A biological tissue-adhering face 50 is formed on this outer surface 11.

The biological tissue-adhering face 50 will be explained later.

The neck section 20 is a substantially cylindrical member extending in the vertical direction, and a main body section of the neck section 20 has a length of about 22 mm. The neck section 20 gradually thickens from the tip to the distal end. The neck section 20 is made of a biocompatible resin. The biocompatible resin is e.g., a polyetheretherketone (PEEK) resin. The neck section 20 has a head-joining shaft 21 on the tip, and a body-joining shaft 23 on the distal end.

The head-joining shaft 21 is a shaft which is screwed into the head 4, and an M8 size male screw is formed on the head-joining shaft 21. Two lateral holes 22 orthogonally penetrate the head-joining shaft 21.

The body-joining shaft 23 is a substantially elliptical shaft which is fitted into the neck-joining hole 12. A circular lateral hole 24 orthogonally penetrates the body-joining shaft 23.

The body-joining shaft 23 is fitted into the neck-joining hole 12. When the body-joining shaft 23 is fitted into the neck-joining hole 12, the lateral hole 24 and the lateral hole 13 are arranged on the same straight line and communicate with each other.

Cylindrical fixing pins 28 made of a biocompatible resin is inserted (press-fitted) into the lateral hole 24 and the lateral hole 13. The biocompatible resin is e.g., a polyetheretherketone resin.

The leg section 25 is a substantially square pole-shaped member extending in the vertical direction, and a main body section of the leg section 25 has a length of about 90 mm. The leg section 25 gradually narrows from the tip toward the distal end. The leg section 25 is made of a biocompatible resin. The biocompatible resin is e.g., a polyetheretherketone resin. The leg section 25 has a body-joining shaft 26 on the tip.

The body-joining shaft 26 is a substantially elliptical shaft which is fitted into the leg-joining hole 14. A circular lateral hole 27 orthogonally penetrates the body-joining shaft 26.

The body-joining shaft 26 is fitted into the leg-joining hole 14. When the body-joining shaft 26 is fitted into the leg-joining hole 14, the lateral hole 27 and the lateral hole 15 are arranged on the same straight line and communicate with each other.

The fixing pins 28 are inserted (press-fitted) into the lateral hole 27 and the lateral hole 15.

### [Head 4]

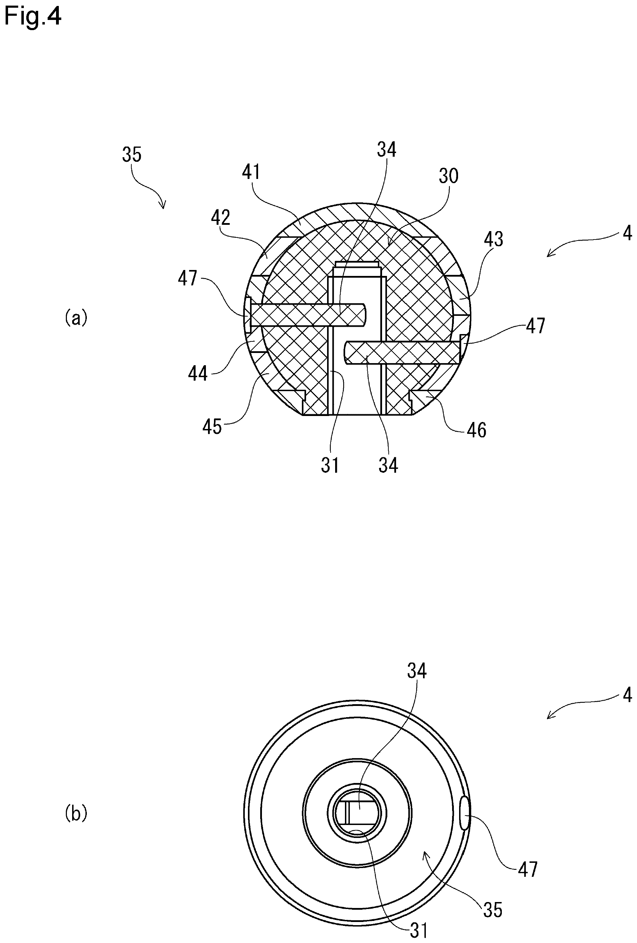

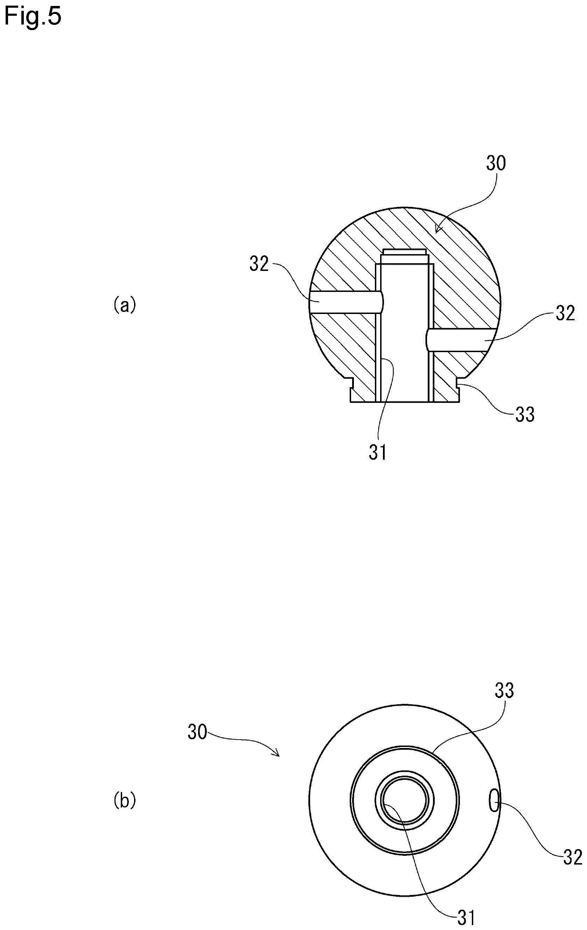

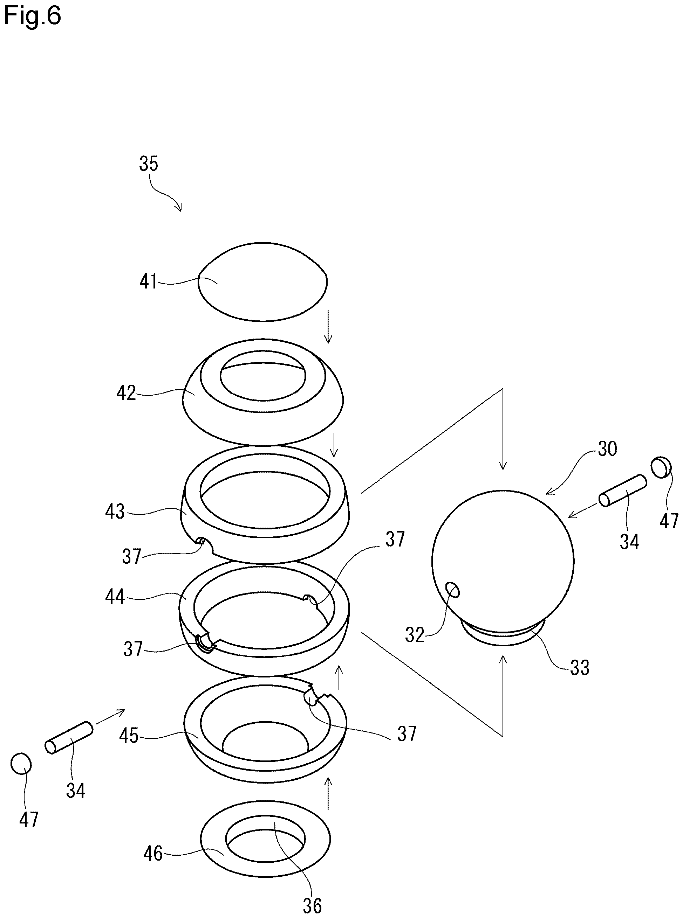

Fig. 4 (a) and (b) are a longitudinal sectional view and a bottom view respectively of the head 4. Fig. 5 (a) and (b) are a longitudinal sectional view and a bottom view respectively of an inner sphere 30. Fig. 6 is an exploded perspective view illustrating the head 4.

The head 4 is a spherical member playing a role of the femoral head and includes an inner sphere 30 and an outer sphere 35.

The inner sphere 30 is a portion joined to the neck section 20 and forms a core of the head 4. The outer sphere 35 is a shell which closely covers the inner sphere 30 and forms the outer surface of the head 4.

The inner sphere 30 is a sphere having an outer diameter of e.g., 25.0 mm. The inner sphere 30 is made of a biocompatible resin. The biocompatible resin is e.g., polyetheretherketone resin.

The inner sphere 30 has a neck-joining hole 31 on the distal end. The neck-joining hole 31 is a hole into which the head-joining shaft 21 is screwed, and an M8 size female screw is formed in the neck-joining hole 31. Two lateral holes 32 orthogonally communicate with the neck-joining hole 31.

When the head-joining shaft 21 is screwed into the neck-joining hole 31, the lateral holes 32 and the lateral holes 22 are arranged on the same straight line and communicate with each other. Fixing pins 34 are inserted (press-fitted) into the lateral holes 32 and the lateral holes 22.

The inner sphere 30 has a fitting shaft portion 33 on an outer edge of the neck-joining hole 31. The fitting shaft portion 33 is a shaft which is to be fitted to the outer sphere 35 (disc body 46), and has an outer diameter of about 14 mm and a length of about 3 mm.

The outer sphere 35 is a sphere having a spherical hollow space. The inner sphere 30 is placed in the inside (hollow space) of the outer sphere 35 with no gap between them. That is, the outer sphere 35 is a hollow spherical shell which closely covers the inner sphere 30. The outer sphere 35 has an outer diameter of e.g., 30.0 mm and a thickness of e.g., 2.5 mm (inner diameter of e.g., 25.0 mm).

The outer sphere 35 has a fitting hole portion 36 which opens to the distal end side and communicates with the hollow space. The fitting hole portion 36 is a hole into which the fitting shaft portion 33 is fitted, and has an inner diameter of about 14 mm.

The outer sphere 35 is made of a biocompatible ceramic. The biocompatible ceramic is e.g., zirconia.

The outer sphere 35 includes six disc bodies 41 to 46 and is configured by stacking them. From the tip to the distal end, the disc bodies 41 to 46 are closely superposed (joined) in this order. The outer sphere 35 (hollow sphere) is configured by arranging the disk bodies 41 to 46 in parallel such that flat faces of the disk bodies are brought into close contact with each other.

The disc bodies 41 to 46 have shapes obtained by slicing the outer sphere 35 in a lateral direction. The sliced shapes refer to shapes obtained by cutting a hollow sphere in parallel in the vertical direction. In other words, the outer sphere 35 includes a plurality of disc bodies 41 to 46 in a spherical segment shape or an end spherical segment shape.

The disc body 41 is a solid (end spherical segment) obtained by cutting a sphere on one flat face. The spherical segment is also referred to as a hemisphere. The disk body 41 has an outer surface and an inner surface which are spherical sectors, and has one circular flat face. The spherical sector is also referred to as a spherical cap or a semispherical surface.

The disk bodies 42 to 46 are solids (spherical segments) obtained by cutting a sphere on two parallel flat faces. An outer surface and an inner surface of each of the disk bodies 42 to 46 are spherical zones. Each of the disk bodies 42 to 45 has two annular flat faces. The disk body 45 has one annular flat face.

The disks 41 and 46 are substantially symmetrical to each other and have a maximum diameter of about 22 mm. The fitting hole portion 36 is formed only on the disk body 46. The disk bodies 42 and 45 are symmetrical to each other and have a maximum diameter of about 28 mm and a minimum diameter of about 22 mm. The disk bodies 43 and 44 are symmetrical to each other and have a maximum diameter of about 30 mm and a minimum diameter of about 28 mm.

The outer sphere 35 has two lateral holes 37. The lateral holes 37 are formed on the disk bodies 43 to 45. When the inner sphere 30 is covered with the outer sphere 35, the lateral holes 37 and the lateral holes 32 overlap and communicate with each other.

A cylindrical fixing pins 34 made of a biocompatible resin are inserted (press-fitted) into the lateral holes 32. The biocompatible resin is e.g., a polyetheretherketone resin.

Disk-shaped caps 47 made of a biocompatible ceramic are fitted into the lateral holes 37. The biocompatible ceramic is e.g., zirconia.

### [Acetabular component 6]

The acetabular component 6 includes the cup 7 and the liner 8.

The cup 7 is a bowl-shaped member implanted into the acetabulum K and is made of a biocompatible ceramic. The biocompatible ceramic is e.g., zirconia. A biological tissue-adhering face 50 is formed on the outer surface of the cup 7.

The liner 8 is a bowl-shaped member fixed inside the cup 7 and is made of e.g., an ultrahigh molecular weight polyethylene resin. The liner 8 slidably supports the head 4 and plays a role of an articular surface.

### [Biological tissue-adhering face 50]

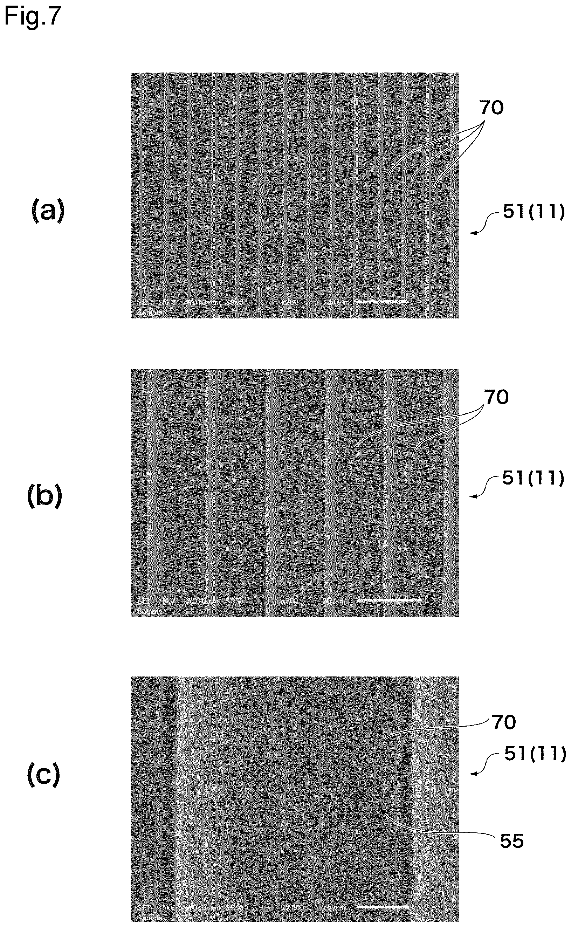

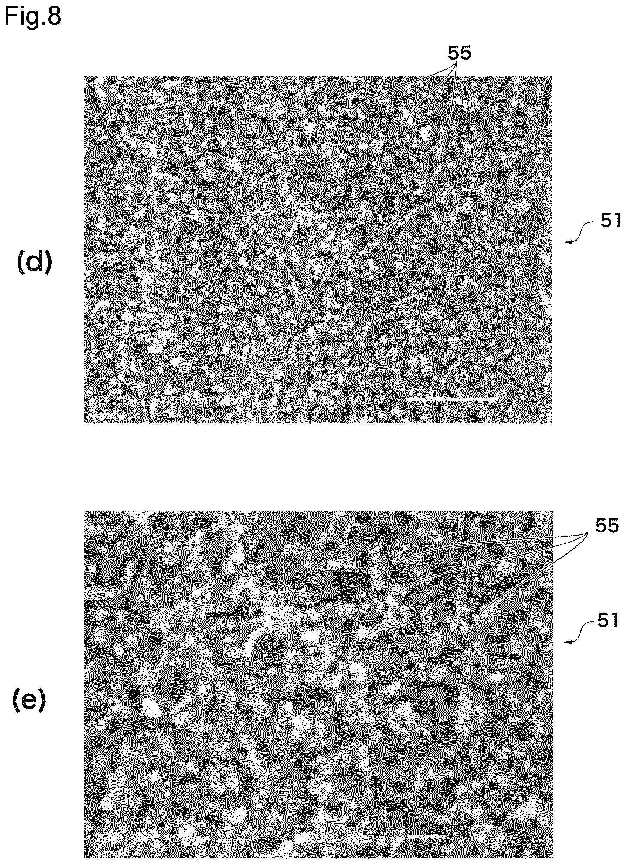

Figures 7 and 8 are photographs of a biological tissue-adhering face 51 taken by SEM at (a) a magnification of 200 times, (b) a magnification of 500 times, (c) a magnification of 2000 times, (d) a magnification of 5000 times, and (e) a magnification of 10000 times.

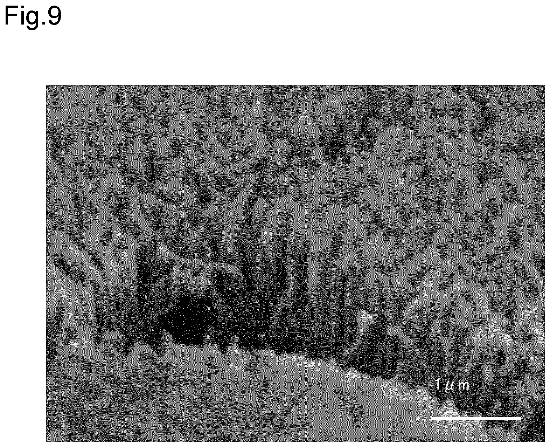

Figure 9 is a reference photograph showing microvilli in a small intestine.

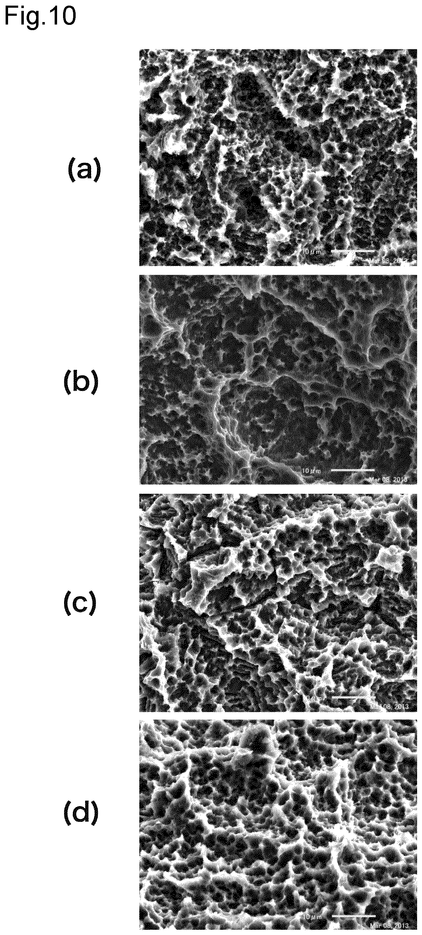

Figure 10 is photographs of outer surfaces of conventional stems taken by SEM (magnification: 2,000 times), including (a) a product manufactured by Company A, (b) a product manufactured by Company B, (c) a product manufactured by Company C, and (d) a product manufactured by Company D.

The biological tissue-adhering face 50 (biological tissue-adhering faces 51 to 54) is formed on the body portion 10. The biological tissue-adhering face 50 is a face for improving osseointegration of the stem 3 to the femur H and is roughened. The biological tissue-adhering face 50 is formed on the outer surface 11 of the body section 10.

The outer surface 11 of the body section 10 closely adheres to the inner face of the narrowing hole Ha of the femur H. Thereby, blood for forming the femur H infiltrates into the biological tissue-adhering face 50. When the biological tissue-adhering face 50 is wetted (infiltrated) with blood, preosteoblasts contained in this blood are fixed to the biological tissue-adhering face 50. Thereby, osseointegration of the body section 10, i.e., the stem 3 is improved.

Hereinafter, some examples of the biological tissue-adhering face 50 (biological tissue-adhering faces 51 to 54) will be explained.

### (Biological tissue-Adhering Face 51)

Numerous fingertip-shaped villi 55 are formed on the biological tissue-adhering face 51. The fingertip shape means a shape with a rounded tip (hemispherical shape) like a fingertip. That is, the tip of the villus 55 is a protrusion with a hemispherical shape on the tip.

The villus 55 is formed so as to have a tip outer diameter of a nanometer size (also referred to as "nanometer order", "nanometer scale", or "nanometer class"). That is, the tip diameter of the villus 55 is 1 nm or more and less than 1000 nm.

The tip diameter of the villus 55 is e.g., 50 nm or more and less than 500 nm. Furthermore, the tip diameter of the villus 55 is e.g., 100 nm or more and less than 300 nm.

Also the biological tissue-adhering face 51 has a three-dimensional surface roughness Sa of a nanometer size (1 nm or more and less than 1000 nm) (arithmetic average height: ISO 25178). For example, the biological tissue-adhering face 51 has a three-dimensional roughness Sa of 500 nm or more and less than 800 nm.

The biological tissue-adhering face 51 has an interface developed area ratio Sdr (ISO 25178) of 0.1 or more and 2.0 or less. The biological tissue-adhering face 51 has the interface developed area ratio Sdr of e.g. 0.5 or more and 1.0 or less.

The villus refers to fine protrusions protruding from a surface of an organ, and exist in small intestine, placenta and the like. The microvillus is also referred to as a soft hair or a soft protrusion.

As shown in Figure 9, in a small intestine, the surfaces of the villi has even more microvilli. The he villus and the microvillus have a fingertip shape. The tip diameter of the microvillus is less than 1 µm. The surface area is significantly increased by villi and microvilli, and absorption and bonding are efficiently and effectively performed.

As described above, the biological tissue-adhering face 51 has numerous villi 55 similar to (closely similar to) the villi or microvilli existing in the biological tissues. For this reason, the biological tissue-adhering face 51 has high bondability and conglutination property with the biological tissue (hard tissues such as bone). That is, the biological tissue-adhering face 51 has an almost ideal shape as a surface configured to be closely bonded to biological tissues and root thereinto.

As shown in Figure 10, the outer surface of the conventional stem is also roughened. These outer surfaces are roughened by etching treatment with hydrochloric acid or the like, or blasting treatment.

Numerous pores are formed on these outer surface, and furthermore numerous protrusions with pointed tips are formed around the pores. The outer surface of the conventional stem has three-dimensional roughness Sa of 2 µm or more.

However, there are found no protrusions with a round tip (fingertip-shaped villi) on any of the outer surface of the conventional stem.

A plurality of large grooves 70 are formed on the biological tissue-adhering face 51.

The large grooves (second grooves) 70 have widths of 10 µm or more and 500 µm or less and juxtaposed. The widths of the large grooves 70 are e.g., 20 µm or more and 100 µm or less. Furthermore, the widths are e.g., 30 µm or more and 50 µm or less.

Depths of the large grooves 70 are 2 µm or more and 100 µm or less. The depths of the large grooves 70 are e.g., 5 µm or more and 10 µm or less.

### (Biological tissue-adhering face 52)

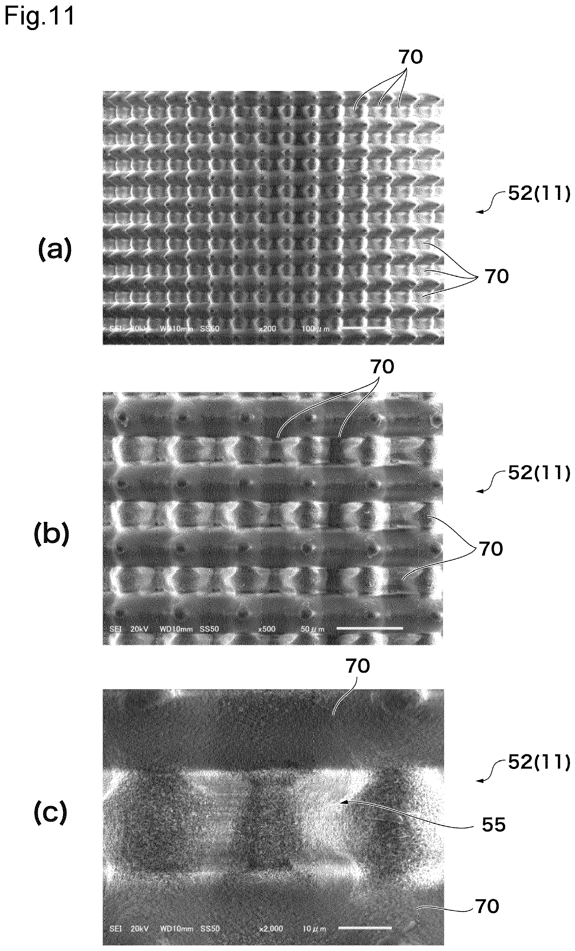

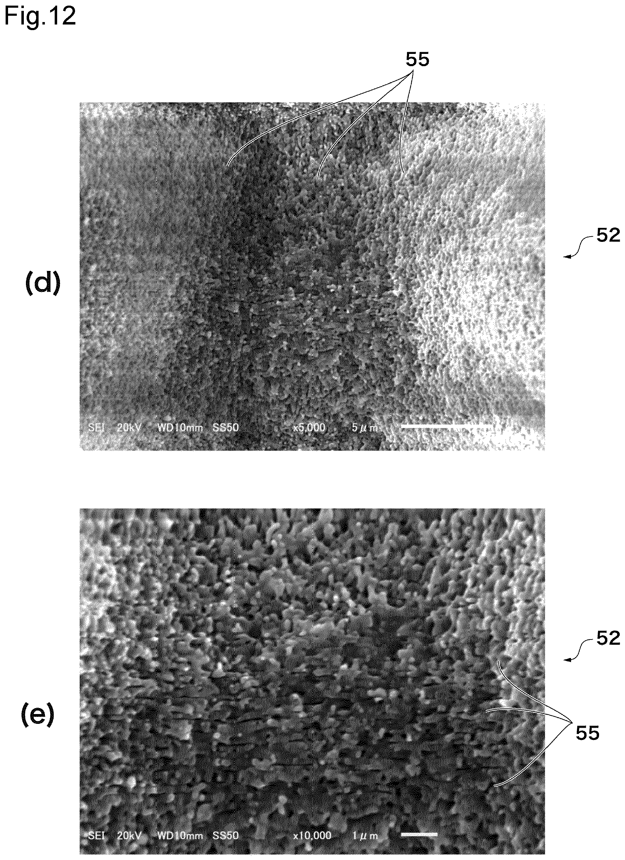

Figures 11 and 12 are photographs of a biological tissue-adhering face 52 taken by SEM at (a) a magnification of 200 times, (b) a magnification of 500 times, (c) a magnification of 2,000 times, (d) a magnification of 5,000 times, and (e) a magnification of 10,000 times.

The biological tissue-adhering face 52 may be formed on the outer surface 11 of the body section 10.

Similar to the biological tissue-adhering face 51, numerous fingertip-shaped villi 55 are formed on the biological tissue-adhering face 52. The biological tissue-adhering face 52 has the same three-dimensional roughness Sa and the same interface developed area ratio Sdr as those of the biological tissue-adhering face 51.

A plurality of large grooves 70 are arranged so as to intersect with each other on the biological tissue-adhering face 51. For example, the large grooves 70 juxtaposed lengthwise and the large grooves 70 juxtaposed crosswise intersect with each other. That is, the plurality of large grooves 70 are arranged in a lattice pattern.

An intersection angle between the large grooves 70 intersecting with each other is 60° or larger, e.g., a right angle.

### (Biological tissue-adhering face 53)

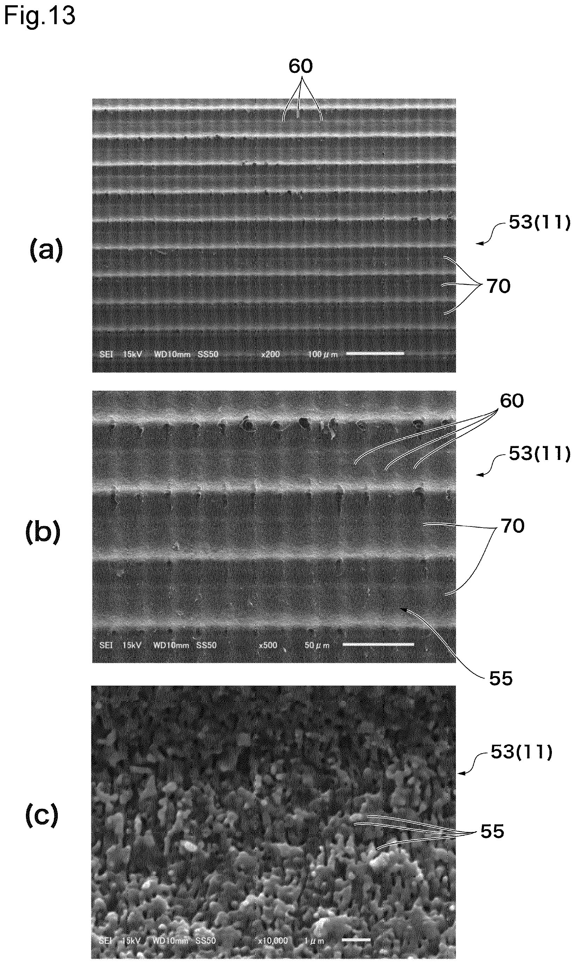

Figure 13 is photographs of a biological tissue-adhering face 53 taken by SEM at (a) a magnification of 200 times, (b) a magnification of 500 times, and (c) a magnification of 10,000 times.

The biological tissue-adhering face 53 may be formed on the outer surface 11 of the body section 10.

Similar to the biological tissue-adhering faces 51 and 52, numerous fingertip-shaped villi 55 are formed on the biological tissue-adhering face 53. The biological tissue-adhering face 53 has the same three-dimensional roughness Sa and the same interface developed area ratio Sdr as those of the biological tissue-adhering faces 51, 52.

A plurality of small grooves 60 and a plurality of large grooves 70 are formed on the biological tissue-adhering face 53. The juxtaposed small grooves 60 and the juxtaposed large grooves 70 crosswise intersect with each other.

An intersection angle between the small grooves 60 and large grooves 70 is 60° or larger, e.g., a right angle.

The small grooves (first grooves) 60 have a width of 1 µm or more and 50 µm or less, and are juxtaposed. The small grooves 60 have a width of e.g., 1 µm or more and 20 µm or less, and furthermore e.g., 1 µm or more and 10 µm or less.

The small grooves 60 have a depth of 0.1 µm or more and 20 µm or less, e.g., 0.1 µm or more and to 5 µm or less.

### (Biological tissue-adhering face 54)

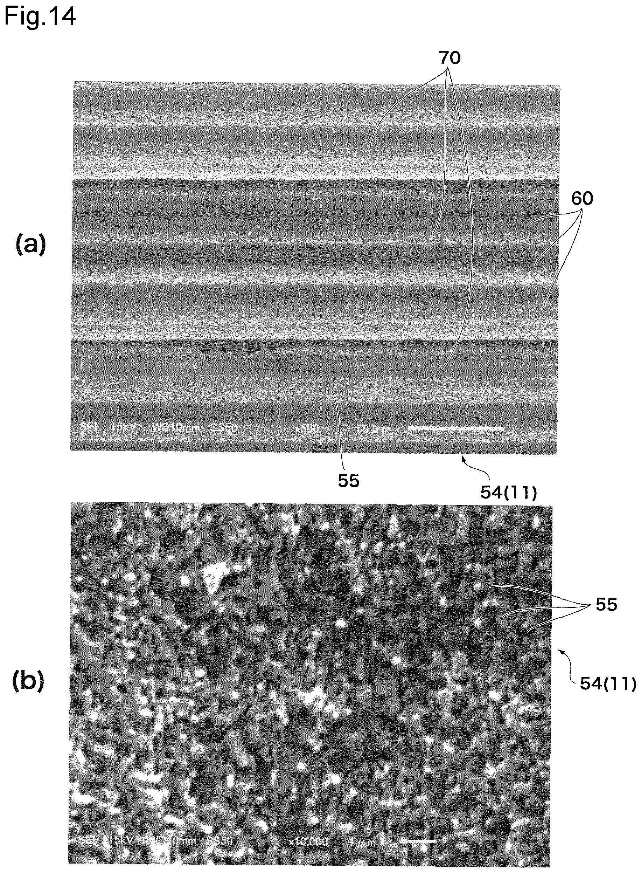

Figure 14 is photographs of a biological tissue-adhering face 54 taken by SEM at (a) a magnification of 500 times, and (b) a magnification of 10,000 times.

The biological tissue-adhering face 54 may be formed on the outer surface 11 of the body section 10.

Similar to the biological tissue-adhering faces 51 to 53, numerous fingertip-shaped villi 55 are formed on the biological tissue-adhering face 54. The biological tissue-adhering face 54 has the same three-dimensional roughness Sa and the same interface developed area ratio Sdr as those of the biological tissue-adhering faces 51 to 53.

A plurality of small grooves 60 and a plurality of large grooves 70 are formed on the biological tissue-adhering face 54. The juxtaposed small grooves 60 and the juxtaposed large grooves 70 extend in the same direction and are superposed. That is, the small grooves 60 are arranged on inner surfaces of the large grooves 70.

For example, the small grooves 60 and the large grooves 70 are parallel to each other. Also, an intersection angle between the small grooves 60 and large grooves 70 may be 30° or less.

### [Production method of artificial hip joint 1]

The body section 10, the outer sphere 35 (disc bodies 41 to 46), the cup 7, and the like are formed of a biocompatible ceramic containing zirconia (zirconium oxide) and the like as main components. Hereinafter, these members are collectively referred to as ceramic parts.

The neck section 20, the leg section 25, the inner sphere 30, and the like are made of a biocompatible resin such as a polyetheretherketone resin. The liner 8 is formed of a polyethylene resin. Hereinafter, these members are collectively referred to as resin parts.

### (Manufacturing Process of Ceramic Parts)

The manufacturing process of the ceramic parts includes a molding step, a sintering step, a processing step, a roughening step (forming step of the biological tissue-adhering face 50), and a washing step.

In the molding step, a pellet containing a zirconia powder is injection-molded to obtain a zirconia compact (ceramic compact). That is, a compact of the body section 10 and the like is obtained.

Next, in the sintering step, the zirconia compact is subjected to presintering and main sintering to obtain a sintered zirconia compact (sintered ceramic compact). That is, a sintered compact of the body section 10 and the like is obtained.

Subsequently, in the processing step, the sintered zirconia compact is subjected to cutting, polishing, and the like to form the neck-joining hole 12 and the like. The outer sphere 35 (disk bodies 41 to 46) is temporarily assembled, and the outer surface of the outer sphere 35 is polished to form a spherical surface with no level difference.

Next, in the roughening step, the outer surface of the sintered zirconia compact (the body section 10, the cup 7) is irradiated with a laser beam to form the biological tissue-adhering face 50.

For the laser beam, a laser beam of an ultrashort pulse laser is used. A laser beam of a picosecond laser or a femtosecond laser can be used.

The ultrashort pulse laser is an extremely short pulse laser with a pulse width (time width) ranging several picoseconds to several femtoseconds. A several-picosecond laser is a laser with a pulse width of one trillionth of a second. A femtosecond laser is a laser with a pulse width of one-quadrillionth of a second.

When the sintered zirconia compact is irradiated with a laser beam of a femtosecond laser or the like, an irradiated face is non-thermally processed (laser nonthermal processing).

The nonthermal processing refers to a process that the sintered compact is irradiated with a laser beam under atmospheric pressure (in air containing moisture) to instantaneously melt, evaporate and scatter the sintered compact. Since the melted site is instantaneously evaporated, scattered and removed, thermal influence (thermal damage) to the surroundings of the processed portion is extremely small. For the nonthermal processing, a pulsed laser with a high laser beam output (peak power or energy density) is used.

The outer surface of the sintered zirconia compact is non-thermally processed with a laser beam to form the biological tissue-adhering face 50 having numerous villi 55. The morphology such as the size of the villi 55 can be changed by adjusting the output, the irradiation time, and the like of the laser beam.

The small grooves 60 and the large grooves 70 are dug into the outer surface of the sintered zirconia compact by scanning the surface while emitting the laser beam.

The machining width (light diameter) of the laser beam can be changed by adjusting the output of the laser beam. That is, the widths and the depths of the small grooves 60 and large grooves 70 can be changed by adjusting the output (machining width) of the laser beam. Also, the widths and the depths of the small grooves 60 and large grooves 70 can be changed depending on the number of irradiation on the same portion, the scanning speed, the laser beam output, and the like.

When the small grooves 60 and the large grooves 70 are individually formed, first, the large grooves 70 are arranged, and then the small grooves 60 are formed.

When the small grooves 60 are arranged in a lattice pattern, or the large grooves 70 are arranged in a lattice pattern, or the small grooves 60 and the large grooves 70 are formed in a lattice pattern, scanning is carried out with a laser beam in two intersecting (orthogonal) directions. At this time, the intersection angle between the bidirectional scanning is an intersection angle between the small grooves 60, between the large grooves 70, or between the small grooves 60 and the large grooves 70.

When the outer surface of the sintered zirconia compact is scraped with a laser beam to form the large grooves 70 and the small grooves 60, the villi 55 are simultaneously formed on the inner surfaces of the large grooves 70 and the small grooves 60. That is, the biological tissue-adhering face 50 is formed simultaneously with the large grooves 70 and the small grooves 60.

When the biological tissue-adhering face 50 is formed, the laser beam output may be varied. That is, the outer surfaces of the body section 10 and the like do not necessarily have the same surface texture (surface roughness) over the entire surface.

Finally, the washing step is performed. In the washing step, a zirconia powder, contaminants, oil, and the like adhering to the sintered zirconia compact are washed away.

In this way, the ceramic parts are manufactured.

### (Manufacturing Process of Resin Parts)

The manufacturing process of the resin pars includes a molding step, a processing step, and a washing step.

In the molding step, a resin compact is obtained by injection molding or the like.

Subsequently, in the processing step, the resin compact is subjected to cutting, polishing, and the like to form the head-joining shaft 21 and the like.

Finally, in the washing step, a resin powder, contaminants, oil, and the like adhering to the resin compact are washed away.

In this way, the resin parts are manufactured.

### (Assembly of Femoral Component 2)

After the ceramic parts and the resin parts are completed, the femoral component 2 is assembled. In the assembly step, first, each of the stem 3 and the head 4 is assembled.

In assembling the stem 3, the neck section 20 and the leg section 25 are joined to the body section 10.

The body-joining shaft 23 is fitted into the neck-joining hole 12. The fixing pins 28 are press-fitted into the lateral holes 13 and 24. Thereby, the neck section 20 is fixed to the body section 10.

The body-joining shaft 26 is fitted into the leg-joining hole 14. The fixing pins 28 are press-fitted into the lateral holes 15 and 27. Thereby, the leg section 25 is fixed to the body section 10.

In assembling the head 4, the outer sphere 35 (disk bodies 41 to 46) is joined to the inner sphere 30. These members are stuck using an adhesive or the like.

The disc bodies 44 to 46 are fitted in this order into the distal end side of the inner sphere 30. The disk bodies 43 to 41 are fitted in this order into the tip side of the inner sphere 30. The fitting hole portion 36 is fitted into the fitting shaft portion 33 to communicate the lateral holes 37 with the lateral holes 22.

Subsequently, the stem 3 and the head 4 are joined.

The head-joining shaft 21 is screwed into the neck-joining hole 31. The fixing pins 34 are press-fitted into the lateral holes 22 and 32. Furthermore, the caps 47 are fitted into the lateral holes 37. The caps 47 are stuck using an adhesive.

Thereby, the head 4 is fixed to the stem 3. That is, assemble of the femoral component 2 is completed.

### (γ-Radiation Sterilization Step, Heating Step)

Finally, a γ-radiation sterilization step is performed. A heating step is additionally performed as necessary.

In the γ-radiation sterilization step, the femoral component 2, the cup 7, and the liner 8 are irradiated with γ-ray for sterilization.

Through the γ-radiation sterilization step, the ceramic parts are dark-browned. Thus, a heating step may be performed. In this heating step, the femoral component 2 and the cup 7 are reheated at 100 to 300°C. Thereby, the color of the ceramic parts is restored (whitened).

Since the polyetheretherketone resin has a heat resistance to temperature of 300°C or higher, it is not deformed or denatured through the heating step.

In this way, the femoral component 2 and the acetabular component 6 (cup 7, liner 8) are manufactured.

In the manufacturing process of the ceramic parts, the roughening step (formation of the biological tissue-adhering face 50) is not necessarily carried out after the main sintering.

The presintering of the ceramic compact may be followed by the roughening step of the sintered ceramic compact. In this case, the main sintering is performed thereafter. Through this main sintering, the sintered ceramic compact contracts. Thus, in consideration of the contraction of the sintered ceramic compact, the ceramic part is formed larger. Thereby, a ceramic part (biological tissue-adhering face 50) having the same shape as in the case of roughening step after the main sintering can be obtained.

### [Replacement Surgery Method of Hip Prosthesis 1]

The hip prosthesis 1 is attached to a patient in accordance with the following procedure. That is, the hip prosthesis replacement surgery includes incision, removal of a femoral head, treatment of an acetabular cartridge, placement of an acetabular component, treatment of a femoral medullary cavity, placement of a femoral component, and suture.

First, in the incision, a lateral-side skin of a hip joint is ground to expose the hip joint.

Next, in the removal of the femoral head, the femur is dislocated from the acetabular cartridge to expose the acetabular cartridge and the femoral head. The femoral head is cut off and removed.

In the treatment of the acetabular cartridge, the acetabular cartridge is cut using a dedicated jig to form a hole into which the cup 7 is fitted.

In the placement of the femoral component, the cup 7 is fitted into the hole formed on the acetabular cartridge, and furthermore the liner 8 is fitted into the cup 7 and fixed.

In the treatment of the femoral medullary cavity, a dedicated jig is inserted into a position where the femoral head has been cut off, to remove a part of a cancellous bone and a bone marrow. The narrowing hole Ha into which the stem 3 is fitted is formed. The narrowing hole Ha is formed so as to gradually narrow from an epiphysis region toward a diaphysis region.

In the placement of the femoral component, the femoral component 2 is inserted (implanted) into the narrowing hole Ha. The head 4 is fitted into the liner 8. That is, the femoral component 2 and the acetabular component 6 are slidably joined.

Finally, the wound is sutured and closed.

### [Effect of Hip Prosthesis 1]

The stem 3 includes the body section 10, the neck section 20, and the leg section 25. The body section 10 is made of zirconia which is a biocompatible ceramic. The neck section 20 and the leg section 25 are made of a polyetheretherketone resin which is a biocompatible resin. Thereby, the stem 3 can be lightened compared to the conventional stem. The weight is about 70 to 80% of the weight of the conventional stem. Consequently, leadenness, uncomfortable feeling, dull pain, pain, or the like of the femoral region can be reduced.

When a high load applied to the stem is transmitted to the diaphysis region (distal side), the bone may be broken. In particular, the diaphysis region is vulnerable to shear force (torsional load).

On the other hand, in the stem 3, the load is transmitted to the epiphysis region (proximal side) through the body section 10. This is because the body section 10 is more firmly osseointegrated to the femur than the leg section 25.

An entire length of the body section 10 is 40% or more and 60% or less of an entire length of the leg section 25. Thus, most of the load applied to the stem 3 is transmitted to the epiphysis region (proximal side) of the femur through the body section 10. That is, the load transmitted to the diaphysis region (distal side) through the leg section 25 is decreased.

Consequently, the load on the femur is sufficiently distributed, and bone atrophy or the like due to stress shielding can be reduced.

Since the neck section 20 which comes into direct contact with a soft tissue is formed of a biocompatible resin, metal allergy and the like can be avoided.

If the neck section is made of a metal, corrosion or metal abrasion powders are generated. The corrosion and metal abrasion powders may lead to looseness or breakage of the stem, resulting in arthralgia, hip joint uncomfortable feeling, tactile tumor, leg edema, femoral neuroparalysis, dislocation, fracture, and deep venous thrombosis.

Since the neck section 20 does not generate corrosion or metal abrasion powders, these problems can be avoided.

The body section 10, the neck section 20, and the leg section 25 are joined (fitted) by shafts and holes and therefore sufficiently absorb at the torsional load. Thus, the stem 3 can distribute and transmit the torsional load to the femur.

The head 4 includes an inner sphere 30 made of a biocompatible resin, and an outer sphere 35 made of a biocompatible ceramic.

Thus, the head 4 can be lightened compared to the conventional femoral component. The weight of the head is about 70% to 80% of the weight of the conventional head. Consequently, leadenness, uncomfortable feeling, dull pain, pain, or the like of the femoral region can be reduced.

The outer sphere 35 is assembled by superposing (joining) the sliced disc bodies 41 to 46. Thus, the outer sphere 35 is preferably configured as a hollow spherical shell having a thin thickness. In particular, even a biocompatible ceramic can preferably configure a hollow spherical shell having a thin thickness.

Since the femoral component 2 (stem 3) and the acetabular component 6 (cup 7) have the biological tissue-adhering faces 50 to 54, preferable osseointegration can be achieved.

The stem 3 has the biological tissue-adhering face 50 on the outer surface 11 of the body section 10. The biological tissue-adhering face 50 has numerous fingertip-shaped villi 55 formed by laser nonthermal processing.

The biological tissue-adhering face 50 is provided to enhance proliferation of preosteoblasts and osteoblasts and shorten the osseointegration period. The biological tissue-adhering face 50 ensures enlargement of the face (area) in contact with blood to facilitate penetration of the preosteoblasts and osteoblasts, so that bone ingrowth is activated and high osseointegration is obtained.

The biological tissue-adhering face 50 has the many small grooves 60 and large grooves 70 to facilitate fixation (anchoring) of the preosteoblasts and the osteoblasts to the biological tissue-adhering face 50. The small grooves 60 and the large grooves 70 are increased in number, varied in size, and made to intersect with each other, so that a dynamic stimulation (mechanical stress) can be provided to the preosteoblasts and the osteoblasts to enhance differentiation into osteoblasts.

In particular, the biological tissue-adhering face 50 has a plurality of ups and downs with different sizes. That is, it has the ups and downs with a nanometer size (villi 55), a size of 1 to 9 micrometers (small grooves 60), and a meso-scale size (large grooves 70). Thus, the biological tissue-adhering face 50 can effectively apply a mechanical stimulation to the preosteoblasts.

Consequently, the bond between the stem 3 (body section 10) and the femur H becomes firm compared to before.

The biological tissue-adhering face 50 is formed on a region which closely adheres to the bone. The biological tissue-adhering face 50 may be formed on one or plural regions as long as the regions can closely adheres to the bone. The region to be roughened may have any area. The biological tissue-adhering face 50 may be formed over the whole outer surface 11.

The small grooves 60 and the large grooves 70 are formed so as to have semicircular cross sections. The cross-sectional shapes may be a triangle (isosceles triangle), a rectangle, or the like.

Each of the small groove 60 and large groove 70 may have a uniform width in the longitudinal direction, or may have different widths, and furthermore may have a uniform depth in the longitudinal direction, or may have different depths.

The plurality of small grooves 60 and large grooves 70 may have a uniform width, or may have different widths, and furthermore may have a uniform depth, or may have different depths.

The numbers of the small grooves 60 and large grooves 70 are arbitrary. The small grooves 60 and large grooves 70 may be formed not only in a straight line but also in a curve line. Preferably, the adjacent small grooves 60 and the adjacent large grooves 70 are arranged at an interval as small as possible. The extending direction of the small grooves 60 and the large grooves 70 forms any angle with respect to the vertical direction of the body section 10.

The biological tissue-adhering faces 51 to 54 may be mixedly formed on the outer surface 11. It is sufficient that any one or more of the biological tissue-adhering surfaces 51 to 54 are formed.

For the biological tissue-adhering face 50, the arrangements of the small grooves 60 and the large grooves 70 can be arbitrarily set. For example, the plurality of large grooves 70 may be arranged in a lattice pattern, on which furthermore the plurality of small grooves 60 may be arranged in a lattice pattern (in an intersecting or superposed manner). The plurality of large grooves 70 may be juxtaposed, on which furthermore the plurality of small grooves 60 may be arranged in a lattice pattern (in an intersecting or superposed manner). Only the plurality of small grooves 60 may be arranged in a lattice pattern.

The joining of the hip prosthesis 1 to a human body is more strengthened by forming the biological tissue-adhering face 50 on the body section 10 and the like.

The body section 10 of the stem 3 and the outer sphere 35 of the head 4 are made of a biocompatible ceramic zirconia.

The body section 10 and the outer sphere 35 may be formed of titanium (pure titanium, titanium alloy) which is a biocompatible metal. Among metals, titanium (pure titanium, titanium alloy) characteristically causes very little allergy and very little adverse effect on a body. It is believed that only a very few people develop metal allergies to titanium.

However, since the stem 3 (hip prosthesis 1) comes into direct contact with a mucosa of a human body, it is highly necessary to avoid a risk of a metal allergy. In particular, since the outer sphere 35 of the head 4 is worn by friction, ionization easily occurs when the outer sphere 35 is made of a metal. For this reason, a person having no metal allergy may develop a metal allergy several years or several decades later. Furthermore, a metal allergy may suddenly develop one day due to aging, changes in physical condition, or the like even in a person who have had no allergy in the past.

Consequently, it is most preferable that no metal is used for the risk of metal allergy in association with long-term use of the stem 3 (hip prosthesis 1). It is preferable to use a completely metal-free stem 3 (hip prosthesis 1).

Furthermore, sterilization different from the conventional gas sterilization is performed on the stem 3 (hip prosthesis 1). γ-radiation sterilization treatment and heat treatment are performed instead of gas sterilization using an ethylene oxide gas with a high risk. The zirconia stem 3 (hip prosthesis 1) is dark-browned and loses aestheticity by γ-radiation sterilization treatment, but can be whitened by heat treatment.

As described above, the stem (hip prosthesis) requires properties such as radiation resistance, heat resistance, non dust-generating property, durability, and stability. The stem 3 (hip prosthesis 1) makes it possible to avoid the risk of metal allergy and is not denatured even by the γ-radiation sterilization treatment and the heat treatment.

### [Body section 80]

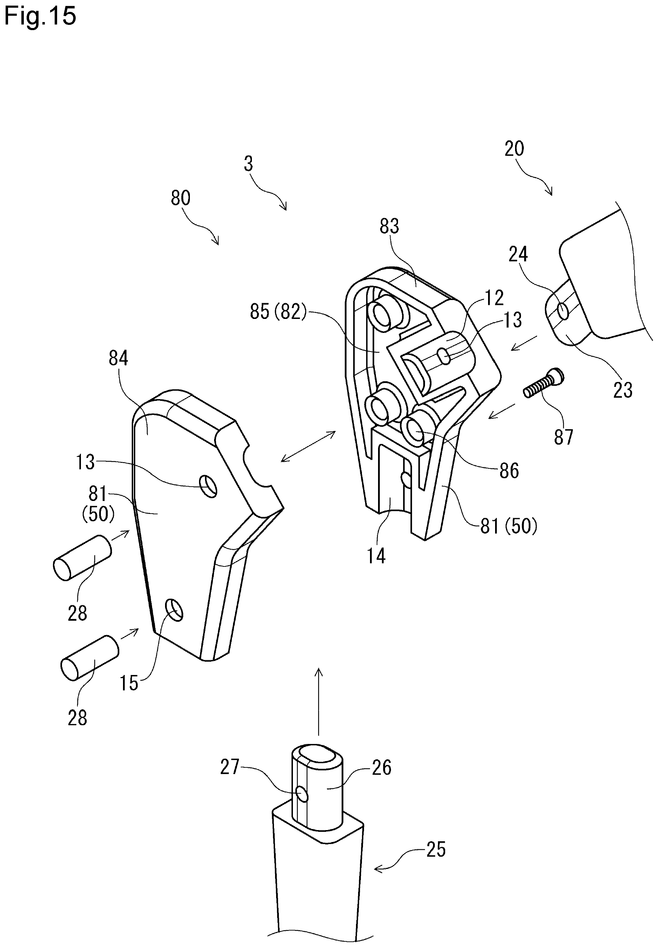

Fig. 15 is an exploded perspective view illustrating a body section 80.

The stem 3 may include the body section 80 instead of the body section 10.

The body section 80 is a modified example (lightened example) of the body section 10 and has substantially the same shape as that of the body section 10. Similarly to the outer surface 11 and the like, the biological tissue-adhering face 50 is formed on an outer surface 81 of the body section 80.

The body section 80 includes a body A83, a body B84, and fixing screws 87.

The body A83 and the body B84 have substantially the same shape as a shape obtained by dividing the body section 10 into two pieces in the anteroposterior direction. The bodies A83 and B84 are substantially symmetrical to each other, and each of them has a concave portion 85 and three bosses 86 on the inner face side.

The body A83 and the body B84 are made of a biocompatible ceramic. The biocompatible ceramic is e.g., zirconia.

The fixing screws 87 are made of a biocompatible resin. The biocompatible resin is e.g., a polyetheretherketone resin.

The body A83 and the body B84 are superposed, and the bosses 86 in close contact with each other are fastened with the fixing screws 87 to assemble the body section 80.

Thereby, the body section 80 has a closed hollow space 82 in the inner side. This closed hollow space 82 is formed by superposing the concave portions 85. The closed hollow space 82 accounts for 40% or more and 60% or less of an entire volume of the body section 80 (including the volume of the closed hollow space 82). Thus, the body section 80 is lightened compared to body section 10.

### [Stem 103]

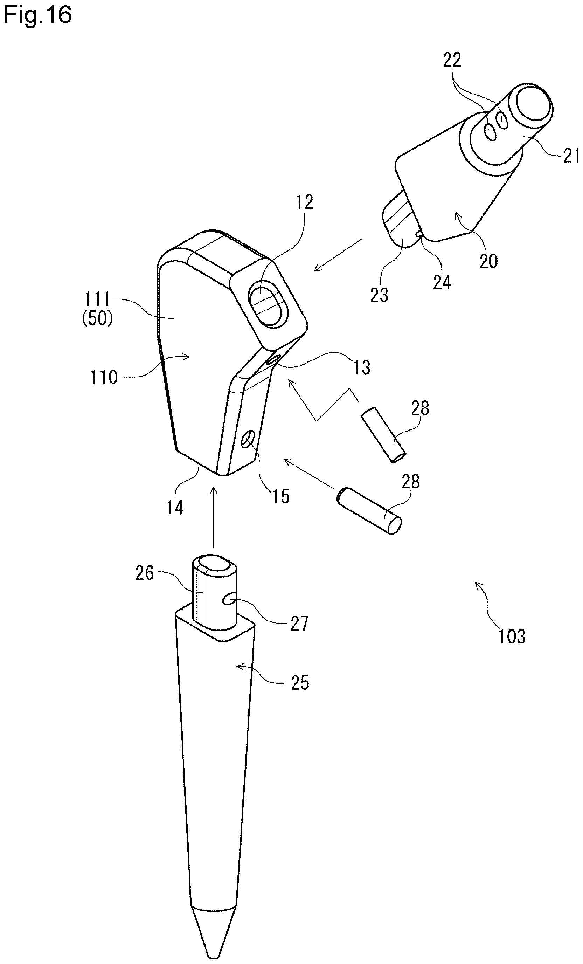

Fig. 16 is an exploded perspective view illustrating a stem 103 according to the second embodiment of the present invention. The members and the like in the second embodiment which are the same as those in the first embodiment are given the same symbols as those in the first embodiment, and explanation thereof is omitted.

The stem 103 includes a body section 110, the neck section 20, and the leg section 25, which are made only of a biocompatible resin. That is, the body section 110 is formed only of a biocompatible resin. The biocompatible resin is e.g., a polyetheretherketone resin.

The body section 110 has the same shape as that of the body section 10. Similarly to the outer surface 11 and the like, an outer surface 111 of the body section 110 has the biological tissue-adhering face 50.

Since the body section 110 is made of a biocompatible resin, it is significantly lightened compared to the body section 10. Thereby, the stem 103 can be significantly lightened compared to the conventional stem. The weight is about 50 to 60% of the weight of the conventional stem. Consequently, leadenness, uncomfortable feeling, dull pain, pain, or the like of the femoral region can be reduced.

Since the biological tissue-adhering face 50 is formed on the outer surface 111 of the body section 110, preferable osseointegration can be achieved

The body section 110, the neck section 20, and the leg section 25 are joined (fitted) by shafts and holes and therefore sufficiently absorb at the torsional load. Thus, despite being formed only of a bioconpatible resin, the stem 103 does not broken by a torsional load and can distribute and transmit the torsional load to the femur.

As described above, the body section 110 acts like the body section 10. Thus, the stem 103 exhibits the same effect as that of the stem 3.

The technical scope of the present invention is not limited to the aforementioned embodiments. The technical scope includes various changes added to the aforementioned embodiments without departing from the gist of the present invention. The specific materials, layer configurations, and the like described in the embodiments are merely examples and can be appropriately changed.

In the aforementioned embodiments, although the zirconia (zirconium oxide) has been explained as the biocompatible ceramic material for forming the body section 10 and the like, the present invention is not limited thereto. The biocompatible ceramic material may be a combination of the zirconia with carbon, resin, glass, or the like. The zirconia (zirconium oxide) only needs to be contained in a volume ratio of 50% or higher relative to the ceramic part such as the body section 10. For example, the zirconia (zirconium oxide) is contained in a volume ratio of 90% or higher relative to the ceramic part such as the body section 10.

As a biocompatible ceramic material, alumina (aluminum oxide), yttrium oxide, hafnium oxide, silicone oxide, magnesium oxide, cerium oxide, or the like may be adopted.

The body section 10 and the like may be formed of not only the biocompatible ceramic but also a biocompatible metal. As the biocompatible metal, copper, titanium, a titanium alloy, a cobalt-chromium alloy, or the like may be adopted. The biocompatible resin may be a composite material or the like.

The stem 3 (neck section 20) and the head 4 are not necessarily fastened with the screws (screwed). The stem 3 and the head 4 may be joined with a so-called snap-fit or the like. They may be stuck using an adhesive.

The stem 3 and the head 4 are not necessarily joined during manufacturing. The stem 3 and the head 4 may be joined during surgery.

The inner sphere 30 and the outer sphere 35 are not necessarily stuck using an adhesive. The inner sphere 30 and the outer sphere 35 may be fixed to each other with a so-called snap-fit or the like. They may be fixed using a snap ring, a screw, a nail, or the like.

### Reference Numerals

- 1: hip prosthesis
- 2: femoral component
- 3: stem
- 4: head
- 6: acetabular component
- 7: cup
- 8: liner
- 10: body section
- 11: outer surface
- 20: neck section
- 25: leg section
- 30: inner sphere
- 35: outer sphere
- 41 to 46: disc body
- 50 to 54: biological tissue-adhering face
- 55: villus
- 60: small groove (first groove)
- 70: large groove (second groove)
- 80: body section
- 81: outer surface
- 103: stem
- 110: body section
- 111: outer surface
- H: femur
- Ha: narrowing hole
- K: acetabulum

## Claims

1. A stem (3) comprising:
a body section (10) which is inserted into a narrowing hole (Ha) formed in a femur (H) and osseointegrated;
a neck section (20) which is joined to a tip of the body section (10) and protrudes from the narrowing hole (Ha) to transmit a load from an acetabular side to the body section (10); and
a leg section (25) which is joined to a distal end of the body section (10) to hold a posture of the body section (10),
wherein the neck section (20) and the leg section (25) are made of a biocompatible resin, and
the body section (10) is made of a biocompatible metal, a biocompatible ceramic, or a biocompatible resin.

2. The stem (3) according to claim 1, wherein the biocompatible resin is a polyetheretherketone resin.

3. The stem (3) according to claim 1 or 2, wherein the biocompatible metal is a titanium alloy or a cobalt-chromium alloy.

4. The stem (3) according to claim 1 or 2, wherein the biocompatible ceramic contains zirconia.

5. The stem (3) according to any one of claims 1 to 4, wherein an entire length of the body section (10) is 40% or more and 60% or less of an entire length of the leg section (25).

6. The stem (3) according to any one of claims 1 to 5, wherein the body section (10) has a hollow space (82) accounting for 40% or more and 60% or less of an entire volume.

7. The stem (3) according to any one of claims 1 to 6, wherein an outer surface (11) of the body section (10) has a biological tissue-adhering face (50 to 54) on which numerous fingertip-shaped villi (55) are formed.

8. The stem (3) according to claim 7, wherein the villi on the biological tissue-adhering face (50 to 54) have a tip diameter in an order of nanometers.

9. The stem (3) according to claim 8, wherein the villi on the biological tissue-adhering face (50 to 54) have a tip diameter of 50 nm or more and less than 500 nm.

10. The stem (3) according to any one of claims 7 to 9, wherein the biological tissue-adhering face (50 to 54) has a three-dimensional surface roughness Sa in an order of nanometers.

11. The stem (3) according to any one of claims 7 to 10, wherein an interface of the biological tissue-adhering face (50 to 54) has a developed area ratio Sdr of 0.1 or more and 2.0 or less.

12. The stem (3) according to any one of claims 7 to 11, wherein the biological tissue-adhering face (50 to 54) has many first grooves (60) having a width of 1 µm or more and 50 µm or less.

13. The stem (3) according to claim 12, wherein the first grooves (60) have a depth of 0.1 µm or more and 10 µm or less.

14. The stem (3) according to any one of claims 7 to 13, wherein the biological tissue-adhering face (50 to 54) has many second grooves (70) having a width of 10 µm or more and 500 µm or less.

15. The stem (3) according to claim 14, wherein the second grooves (70) have a depth of 2 µm or more and 100 µm or less.

16. The stem (3) according to any one of claims 7 to 15, wherein the biological tissue-adhering face (50 to 54) is formed through a laser nonthermal processing carried out by emitting a laser beam in air.

17. A femoral component (2) comprising:
the stem (3) according to any one of claims 1 to 16; and
a spherical head (4) attached to a tip of the stem (3) and receiving a load from an acetabular side,
wherein the head (4) comprises:
an inner sphere (30) made of a biocompatible resin and joined to the neck section (20); and
an outer sphere (35) made of a biocompatible metal or a biocompatible ceramic and surrounding the inner sphere (30).

18. The femoral component (2) according to claim 17, wherein the outer sphere (35) comprises a plurality of disc bodies (41 to 46) having a spherical segment shape or an end spherical segment shape, and is configured by joining the plurality of disc bodies (41 to 46).

19. A hip prosthesis (1) comprising:
the femoral component (2) according to claim 17 or 18; and
an acetabular component (6) having a cup (7) and a liner (8) and in close contact with the femoral component (2).

20. The hip prosthesis (1) according to claim 19, wherein an outer surface of the cup (7) has a biological tissue-adhering face (50 to 54) on which numerous fingertip-shaped villi (55) are formed.
